# EUROPÄISCHE PATENTSCHRIFT

(11) **EP 0 742 702 B1**
(45) Veröffentlichungstag und Bekanntmachung des Hinweises auf die Patenterteilung: **06.05.1998**
(21) Anmeldenummer: 95907550.8
(22) Anmeldetag: 02.02.1995
(51) Int. Cl.: A61F 2/16

(54) **INTRAOKULARE LINSENANORDNUNG ZUR ASTIGMATISMUSKORREKTUR**
INTRA-OCULAR LENS ARRANGEMENT FOR CORRECTING ASTIGMATISM
AGENCEMENT DE LENTILLES INTRAOCULAIRES POUR CORRECTION DE L'ASTIGMATISME

(30) Priorität: 03.02.1994 DE 4403326
(43) Veröffentlichungstag der Anmeldung: 20.11.1996
(73) Patentinhaber: Koch, Hans-Reinhard, 53177 Bonn (DE)
(72) Erfinder: Koch, Hans-Reinhard, 53177 Bonn (DE)
(74) Vertreter: Haft, von Puttkamer, Berngruber, Czybulka
(86) Internationale Anmeldenummer: DE9500135
(87) Internationale Veröffentlichungsnummer: WO9520926

(56) Entgegenhaltungen:
- EP-A- 0 162 573
- EP-A- 0 337 390
- WO-A-94/07435
- DE-A- 2 548 467
- US-A- 4 575 373
- US-A- 4 994 082
- US-A- 5 222 981

## Beschreibung

Wird im Rahmen der Katarakt-Chirurgie eine getrübte Augenlinse entfernt, so wird diese heute im allgemeinen durch eine implantierte Intraokularlinse ersetzt. Nach vorheriger Berechnung der optischen Konstanten des Auges kann die Intraokularlinse so ausgestaltet werden, daß sie vorher bestehende Refraktionsfehler korrigiert, so z.B. Kurzsichtigkeit, Übersichtigkeit und auch Astigmatismus. Bei entsprechendem Wunsch des Patienten kann die Intraokularlinse so gewählt werden, daß eine bestimmte postoperative Fehlsichtigkeit entsteht, z.B. eine leichte Kurzsichtigkeit, die es dem Patienten möglich macht, in der Nähe ohne Zusatzkorrektur zu sehen.

Während sich die sphärischen Fehlsichtigkeiten, also Kurz- oder Übersichtigkeit, gut korrigieren lassen, ist die Korrektur des Astigmatismus, d.h. einer Stabsichtigkeit, noch heute ein Problem. Dabei ist wichtig zu wissen, daß nicht nur vor der Operation zur Implantation der Intraokularlinse ein Astigmatismus bestehen kann, sondern daß auch durch die Operation, insbesondere durch den Operationsschnitt und die später verheilte Naht dieses Schnittes ein Astigmatismus erzeugt wird. Es ist zwar in der jüngeren Vergangenheit gelungen, durch verbesserte Schnittechniken bei der Implantation das Problem des operationsbedingten Astigmatismus zu verkleinern; eine Beseitigung dieses Astigmatismus ist dagegen nicht möglich. Es ist demnach eine Tatsache, daß jede Kataraktoperation per se ein Astigmatismus erzeugender Eingriff ist, der gegebenenfalls auch zur Verringerung des natürlichen, durch die Geometrie dies Auges bedingten Astigmatismus herangezogen werden kann. Soweit ein Operateur mit der von ihm gewählten Technik einen typischen postoperativen Astigmatismus induziert, könnte zumindest theoretisch der sich nach der Operation einstellende Gesamtastigmatismus korrigiert werden, indem eine Intraokularlinse mit einem den Astigmatismus korrigierenden Kompensationsschliff so eingesetzt wird, daß der sich postoperativ einstellende gesamte Astigmatismus korrigiert wird. Es ist allerdings so, daß die Techniken der Katarakt-Chirurgie so variabel sind, daß eine genaue Voraussage des sich nach der Operation einstellenden Gesamtastigmatismus noch nicht möglich ist. Ein sicheres Beseitigen des postoperativen Astigmatismus ist daher heute noch nicht möglich.

Aus der US-A-5222981 ist eine intraokulare Linsenanordnung mit mehreren Intraokularlinsen bekannt, die längs einer gemeinsamen Achse hintereinander angeordnet sind. Die Linsenanordnung besteht aus einer Basislinse, einer Decklinse und einer dazwischen liegenden Sandwich-Linse. Insbesondere diese Sandwich-Linse kann mit einem Schliff versehen sein, durch den ein Astigmatismus korrigiert werden kann. Die gesamte Linsenanordnung wird in das Auge implantiert, wobei die Sandwich- und die Deckellinse noch nicht mit einem Schliff versehen sein müssen, der zur Behebung einer Fehlsichtigkeit geeignet ist. Erst nach Ausheilen des chirurgischen Eingriffes zur Implantation werden in einem weiteren chirurgischen Eingriff die Deckellinse und die Sandwich-Linse entfernt und durch die Fehlsichtigkeit nunmehr behebende neue Linsen ersetzt. Diese Linsen sind zwar kleiner als die gesamte Linsenanordnung, jedoch ist dieser zweite chirurgische Eingriff fast so gravierend wie der zur Implantation notwendige, da ein Schnitt mit einer Länge von einigen Millimetern durchgeführt werden muß.

Aus der US-A-5133747 ist eine intraokulare Linsenanordnung aus einer Basislinse und einer Decklinse bekannt, die auch zur Korrektur eines Astigmatismus geeignet ist. Bei der Implantation wird zunächst nur die Basislinse implantiert und anschließend in einem kleineren chirurgischen Eingriff die Decklinse auf die Basislinse aufgebracht, z.B. aufgeklebt. Diese Decklinse dient dann zur Korrektur der Fehlsichtigkeit, auch zur Korrektur eines Astigmatismus. Es ist auch hier ein zweiter chirurgischer Eingriff notwendig, auch wenn mit diesem Verfahren der durch die erste chirurgische Operation bei der Implantation verursachte operative Astigmatismus relativ gut kompensiert werden kann. Das Verfahren jedoch, die zusätzliche Decklinse aufzukleben, setzt hohe operative Fertigkeit voraus.

Aus der US-A-4512039 ist eine Intraokularlinse zur Korrektur eines Astigmatismus bekannt, die lediglich aus einer einzigen torisch geschliffenen Intraokularlinse besteht. Zur Reduzierung des postoperativen Astigmatismus wird dieser aus Erfahrungswerten geschätzt und die Intraokularlinse so eingesetzt, daß der Astigmatismus mehr oder minder kompensiert wird. Dieses setzt jedoch voraus, daß der Operateur aufgrund seiner Operationsmethode den zu erwartenden Astigmatismus relativ genau kennt.

Andere relevante Dokumente, die zum Stand der Technik gehören, sind DE-A-2 548 467 und US-A-4 575 373.

Der Erfindung liegt die Aufgabe zugrunde, eine intraokulare Linsenanordnung anzugeben, mit der der postoperativ sich einstellende Astigmatismus optimal korrigiert werden kann.

Ausgehend von einer intraokularen Linsenanordnung der im Oberbegriff des Patentanspruches 1 angegebenen Art ist diese Aufgabe durch die kennzeichnenden Merkmale des Patentanspruches 1 gelöst.

Demgemäß besteht der wesentliche Gedanke der Erfindung darin, eine Linsenanordnung aus zwei auf der gleiche optischen Achse hintereinander angeordnete Intraokularlinsen vorzusehen, die jeweils einen zur Korrektur eines Astigmatismus geeigneten Kompensationsschliff aufweisen und mit der Schlifforientierung gegeneinander verdreht so im Auge einzusetzen, daß der Astigmatismus korrigiert wird. Nach der Verheilung des Operationsschliffes kann dann eine abschließende Korrektur vorgenommen werden, indem die beiden Intraokularlinsen mit einem Werkzeug, das durch einen kleinen, den Astigmatismus nicht verändernden Schnitt in das Auge eingeführt wird, durch eine geringe Verdrehung gegeneinander nachjustiert werden.

Als Kompensationsschliff wird ein Schliff mit der optischen Wirkung einer Zylinderlinse bzw. ein Schliff mit torischer Wirkung verwendet.

Dies kann z.B. ein Zylinderschliff sein, der mit einem sphärischen Schliff zur Kompensation anderer Fehlsichtigkeit kombiniert werden kann, ein torischer Schliff oder ein eine Beugung erzeugender Schliff; vgl. zu letzterem etwa das US-Patent 5100226. Möglich sind auch Intraokularlinsen aus einem flexiblen Material, deren Gestalt zur Erzielung einer optischen Zylinderwirkung durch entsprechende Krafteinwirkung geändert werden kann, vgl. etwa die WO 92/03989, bei der die Form der Linse durch kleine Aktuatoren aus ferromagnetischem Material beeinflußt werden kann, die in einem als Haptik für die Intraokularlinse dienenden Aufnahmering angeordnet sind.

Die Linsenanordnung weist z.B. zwei um die gleiche optische Achse drehbar angeordnete Zylinderlinsen mit sphärischzylindrischem Schnitt auf, deren zylindrische Wirkung, die geringfügig abhängig von dem Implantationsort der Linse im Auge ist, wesentlich gleich ist. Die Dioptrienwerte der beiden Linsen für die zylindrische Wirkung können gleiches oder ungleiches Vorzeichen aufweisen. Werden diese Linsen gegeneinander um die optische Achse so verdreht, daß bei Dioptrienwerten um ungleichen Vorzeichen sich ihre Achsen und stärkstbrechenden Meridiane jeweils gegenüberliegen, bzw. bei gleichen Vorzeichen um 90 Grad gegeneinander verdreht sind, dann kompensieren sich die Zylinderlinsen und es wirkt nur der sphärische Schliff. Werden die Intraokularlinsen aus dieser Stellung heraus um 90 Grad gegeneinander verdreht, so daß bei ungleichen bzw. gleichen Vorzeichen die Achse der einen Linse mit dem stärkstbrechenden Meridian der jeweils anderen Linse koinzidiert bzw. gegenüber diesem um 90 Grad verdreht ist, dann entsteht neben der sphärischen Wirkung eine maximale zylindrische Wirkung entsprechend der Summe der Zylinderwirkungen der beiden Einzellinsen des Systems. Durch Einstellen der beiden Linsen in eine Lage zwischen diesen beiden Extremzuständen können variable Astigmatismuswerte zwischen Null und dem erwähnten Maximalwert eingestellt werden.

Da die Brechkraft eines spaltförmig vor einer Zylinderlinse angeordneten Ausschnittes aus der Optik einer Cosinus-Quadrat-Beziehung gehorcht, haben zwei einander überlagerte Zylinderlinsen daher die Wirkung der Summe der beiden Cosinus-Quadrat-Kurven. Eine solche als Zylinderkompensator zu bezeichnende Linsenanordnung sollte also durch entsprechendes Verdrehen um die optische Achse alle Werte zwischen einem maximalen Zylinderwert entsprechend der Summe der Zylinderbrechkräfte der beiden Linsen und dem Wert Null als anderem Extrem annehmen können.

Die Linsenanordnung wird bei der Implantation auf einen Korrekturwert etwa entsprechend dem erwarteten postoperativen Astigmatismus eingestellt. Sich nach der Operation einstellende Abweichungen könnten dann in einem zweiten Minimaleingriff behoben werden, indem durch einen kleinen Schnitt eine oder beide Intraokularlinsen um die optische Achse verdreht werden. In der klinischen Anwendung könnte eine derartige intraokulare Linsenanordnung als Zylinderkompensator auf verschiedenen Wegen realisiert werden. Vorzugsweise wird er aus zwei sphäro-zylindrischen Linsen zusammengesetzt, wobei diese z.B. in einer gemeinsamen Haptik aufgenommen und mit dieser implantiert werden oder jeweils eine eigene Haptik aufweisen und beide Linsen bei einer Operation in das Auge implantiert werden. Bei dem noch gegenwärtigen Stand der Operationstechnik ist die zweite Variante wohl die bevorzugte Realisation, so daß die Linsen bei einer solche Operation nacheinander in das Auge eingebracht werden, deren Haptik im Kapselsack aufgenommen wird. So könnte z.B. die weiter hinten, dem Augenhintergrund zugewandte Linse eine stärker abgewinkelte Haptik haben, während die vordere, der Hornhaut zugewandte Linse eine plane Haptik oder eine schwächer abgewinkelte Haptik hat. Wichtig ist, daß die Haptikformen so gewählt sind, daß die Linsen im Auge über einen längeren Zeitraum mobilisierbar und drehbar verbleiben, damit man auch später noch entstandene oder geänderte Astigmatismen nachjustieren kann. Am geeignetsten erschient hierbei eine kreisformige Haptik, wobei es eine weitere bevorzugte Ausführungsform ist, einen Ring in den Kapselsackäquator zu implantieren, der für die Haptik der zu implantierenden Intraokularlinsen bzw. der Linsenanordnung als Schiene dient, längs der die Haptiken der Intraokularlinsen verschiebbar sind. Ein ahnliches Aufnahmeprinzip für eine einzige Intraokularlinse ist z.B. aus der DE-A1-40 30 899 bekannt.

Auf jeder Intraokularlinse wird bevorzugt auch noch die Achsrichtung bzw. die Richtung des stärkstbrechenden Meridians gekennzeichnet, damit der Operateur sofort erkennt, in welcher Richtung die Linsen nach der Implantation zu verdrehen sind. Eine solche Kennzeichnung ist auch wichtig, damit gegebenenfalls zu einem späteren Zeitpunkt der Operateur die Stärke des Astigmatismus nachregulieren kann.

Als bevorzugte Ausführungsform einer Kompensations-Linsenanordnung wird ein System aus zwei plankonvexen Linsen vorgeschlagen, deren planen Seiten sich gegenüberstehen und in deren konvexen Seite der Kompensationsschliff vorhanden ist.

Die sphärische Wirkung der Linsenkombination kann generell wahlweise auf die beiden Linsen verteilt oder lediglich auf einer der beiden Linsen aufgebracht werden.

Ebenso ist es möglich, die Linsenkombination mit mehreren Stärken, z.B. für Fern- und Nahsicht auszubilden.

Die Linsen der Linsenanordnung können aus jedem geeigneten harten oder weichen Material hergestellt werden, z.B. aus Polymethylmethacrylat (PMMA), Silicon, Hydrogel, faltbarem Acrylat etc.

Die Erfindung ist in Ausführungsbeispielen anhand der Zeichnung näher erläutert; in dieser stellen dar:
Fig. 1 eine Seitenansicht einer Linsenanordnung gemäß der Erfindung aus zwei hintereinander angeordneten Intraokularlinsen mit jeweils einem Zylinderschliff;
Fig. 2 eine Aufsicht auf die Linsenanordnung gemäß Fig. 1;
Fig. 3 eine schematische perspektivische Ansicht des Linsenteiles einer der Intraokularlinsen der Linsenanordnung gemäß Fig. 1;
Fig. 4 eine Linsenanordnung gemäß der Erfindung aus zwei Intraokularlinsen, deren Haptik in einem gemeinsamen Ring aufgenommen sind;
Fig. 5 einen Schnitt durch den Ring zur Aufnahme der Haptik gemäß Fig. 4 mit angedeutetem perspektivischen Verlauf.

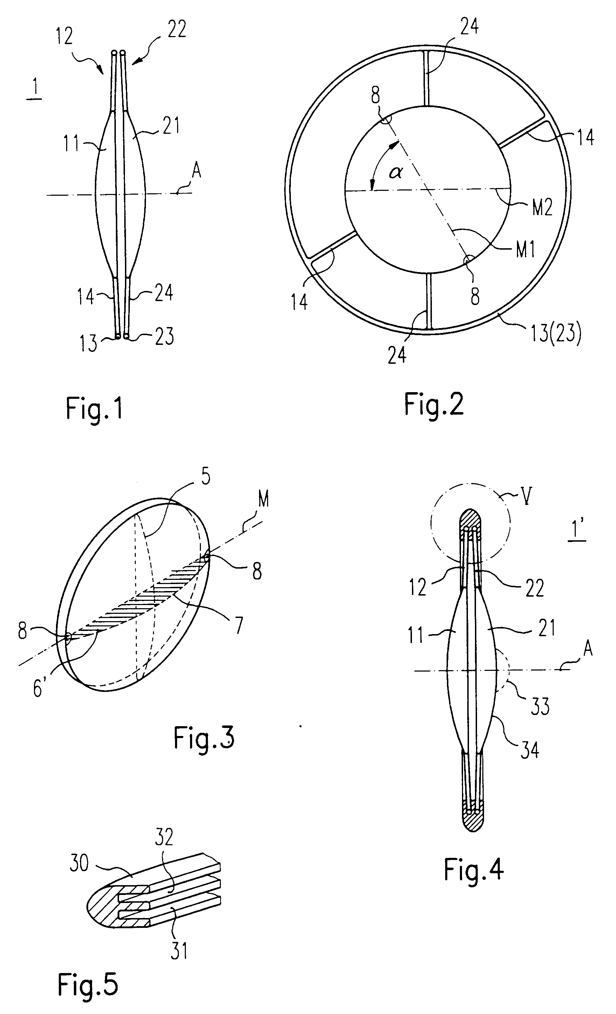

Eine in Fig. 1 gezeigte Linsenanordnung 1 besteht aus einer vorderen plankonvexen Intraokularlinse 11 und einer hinteren ebenfalls plankonvexen Intraokularlinse 21. Die Linsen haben die gemeinsame optische Achse A und sind mit ihren planen Seiten einander zugewandt. Jede Intraokularlinse 11 bzw. 21 weist eine Haptik 12 bzw. 22 auf, die hier jeweils als Ringhaptik mit einem äußeren Ring 13 bzw. 23 und je zwei Stegen 14 bzw. 24 zwischen Linse und Ring ausgebildet ist. Die beiden Haptiken sind radial so ausgerichtet, daß sie im Kapselsack des Auges aufgenommen werden.

Der in Fig. 3 schematisch dargestellte Linsenteil der beiden Intraokularlinsen weist einen angedeuteten sphärischen Schliff 5 und zusätzlich einen ebenfalls angedeuteten Zylinderschliff 6 auf, dessen stärkstbrechender Meridian mit 7 und dessen Meridianrichtung mit M bezeichnet ist.

Die beiden Intraokularlinsen 11 und 21 sind gemäß Fig. 2 so im Auge eingesetzt, daß die Meridianrichtung M1 der Linse 11 mit der Meridianrichtung M2 der Linse 21 einen in Fig. 2 mit α bezeichneten Winkel einschließen. Die jeweilige Meridianrichtung wird durch zwei gegenüberliegende Markierungen 8, z.B. kleine Löcher, Kerben oder sonstige Kennzeichnungen am Rand der Intraokularlinsen angegeben.

Die beiden Intraokularlinsen 11 und 21 konnen zum Einstellen der Meridianrichtung mit einem kleinen Werkzeug z.B. an der Haptik oder - falls vorhanden - an den erwähnten Kerben oder Löchern in ihrer Lage verdreht werden.

Der Winkel α zwischen den Meridianrichtungen M1 und M2 wird nun so eingestellt, daß der zu erwartende postoperative Astigmatismus annähernd kompensiert ist. Eine Nachjustierung kann nach Heilen des Operationsschnittes dann durch ein kleines Manipulationswerkzeug erfolgen, das durch eine Mikroinzision in das Auge eingeführt wird und mit dem der Winkel α zwischen den Meridianrichtungen so justiert wird, daß der Astigmatismus vollständig behoben ist. Eine solche Mikroinzision hat keinen Einfluß auf den Astigmatismus.

In Fig. 4 ist eine modifizierte Ausführungsform einer Linsenanordnung 1' gemäß der Erfindung dargestellt. Die Linsenanordnung weist wiederum eine vordere und eine hintere Intraokularlinse 11 bzw. 21 mit einem sphärisch-zylindrischen Schliff auf, wobei die beiden Haptiken 12 bzw. 22 in einem Ring 30 aufgenommen werden, der im Kapselsack implantiert ist. Der Ring 30 hat längs seines Innenrandes zwei parallele Nuten 31 und 32, wobei in der Nut 31 die Haptik 12 der vorderen Intraokularlinse 11 und in der Nut 32 die Haptik 22 der hinteren Intraokularlinse 21 aufgenommen sind. Die Haptiken der beiden Intraokularlinsen können in den Nuten 31 und 32 des Ringes 30 zur Justierung der Meridianrichtungen verdreht werden.

In Fig. 4 ist gestrichelt für die vordere Linse ein Bereich 33 mit zusätzlichem sphärischen Schliff angedeutet. Dieser Bereich kann für das Sehen in der Nähe dienen, wohingegen dann der entsprechend geschliffene umgebende "äußere" Bereich 34 für das Sehen in der Ferne dient.

Es ist selbstverständlich, daß die gezeigten Linsenanordnungen nur als Ausführungsbeispiele dienen. So können die Linsen andere Formen, andere Schliffarten und andere Haptiken etc. aufweisen, sofern nur sichergestellt ist, daß die Linsen eine bestimmte Ausrichtung des Kompensationsschliffes entsprechend der angegebenen Meridianrichtung aufweisen, so daß durch Einstellen des Winkels zwischen diesen Meridianrichtungen der Astigmatismus korrigiert und feinjustiert werden kann.

Die Linsenanordnung kann an jedem geeigneten weichen oder harten Material hergestellt werden, z.B. PMMA, Silicon, elastischem Hydrogel, faltbarem Acrylat etc.

## Patentansprüche

1. Intraokulare Linsenanordnung (1, 1') aus mehreren, langs einer gemeinsamen optischen Achse hintereinander angeordneten Intraokularlinsen (11, 27) wobei zumindest eine der Intraokularlinsen (11, 21) zur Astigmatismuskorrektur mit einem den Astigmatismus kompensierenden optischen Schliff (6) versehen ist, dadurch gekennzeichnet, daß die Linsenanordnung (1, 1') aus zwei Intraokularlinsen (11, 21) besteht, die jeweils einen zur Korrektur des Astigmatismus geeigneten Kompensationsschliff (6) mit einer linearen bevorzugten Orientierung (M, M1, M2) aufweisen, und beide Intraokularlinsen (11, 21) im implantierten Zustand relativ zueinander um die gemeinsame optische Achse (A) verdrehbar ist.

2. Linsenanordnung nach Anspruch 1, dadurch gekennzeichnet, daß beide Intraokularlinsen (11, 21) in einer gemeinsamen, die Verdrehbarkeit der Intraokularlinsen relativ zueinander zulassenden Haptik aufgenommen sind.

3. Linsenanordnung nach Anspruch 1 oder 2, dadurch gekennzeichnet, daß beide Intraokularlinsen (11, 21) jeweils mit einer Haptik (12, 32) versehen und beide Haptiken (12, 22) in einem gemeinsamen implantierbaren Aufnahmering (30) drehbar aufgenommen sind.

4. Linsenanordnung nach Anspruch 3, dadurch gekennzeichnet, daß der Aufnahmering (30) längs seines Innenrandes zwei parallele Nuten (31, 32) zur Aufnahme der Haptiken (12, 13, 22, 23) aufweist.

5. Linsenanordnung nach einem der vorhergehenden Ansprüche, dadurch gekennzeichnet, daß die Intraokularlinsen (11, 21) mit Kennzeichnungen (8) zur Angabe der bevorzugten linearen Orientierung (M, M1, M2) versehen sind.

6. Linsenanordnung nach einem der vorhergehenden Ansprüche dadurch gekennzeichnet, daß die Intraokularlinsen (11, 21) an ihrem Rand bzw. die Haptik Teile, wie Löcher, Kerben (8) oder Stege (14, 24), zum Drehen der Linsen um ihre optische Achse (A) aufweisen.

7. Linsenanordnung nach einem der vorhergehenden Ansprüche, dadurch gekennzeichnet, daß der Kompensationsschliff zur Astigmatismuskorrektur ein Schliff mit optisch zylindrischer oder torischer Wirkung ist.

8. Linsenanordnung nach einem der vorhergehenden Ansprüche, dadurch gekennzeichnet, daR das Material für die Linsenanordnung ein geeignetes weiches oder hartes Material, wie PMMA, Silicon, Hydrogel, faltbares Acrylat etc. ist.

9. Linsenanordnung nach einem der vorhergehenden Ansprüche, dadurch gekennzeichnet, daß zumindest eine der Linsen (21) der Linsenanordnung eine Mehrstärkenlinse ist.

## Claims

1. Intraocular lens arrangement [1, 1'] consisting of a plurality of intraocular lenses [11, 21] disposed consecutively along a common optical axis, wherein at least one of the intraocular lenses [11, 21] is provided with a ground region [6] for compensating astigmatism, characterised in that the lens arrangement [1, 1'] consists of two intraocular lenses [11, 21], each of which has a compensating ground region [6] for compensating astigmatism and having a linear preferred orientation [M, M1, M2], and both intraocular lenses [11, 21] in the implanted state are rotatable relative to one other about the common optical axis [A].

2. Lens arrangement according to claim 1, characterised in that the two intraocular lenses [11, 21] are held in a common haptic permitting rotation of the intraocular lenses relative to one another.

3. Lens arrangement according to claim 1 or 2, characterised in that the two intraocular lenses [11, 21] are each provided with a haptic [12, 32] and both haptics [12, 22] are rotatably held in a common implanted receiving ring [30].

4. Lens arrangement according to claim 3, characterised in that the receiving ring [30] has along its inner edge two parallel grooves [31, 32] for receiving the haptics [12, 13, 22, 23].

5. Lens arrangement according to one of the preceding claims, characterised in that the intraocular lenses [11, 21] have marks [8] indicating the preferred linear orientation [M, M1, M2].

6. Lens arrangement according to one of the preceding claims, characterised in that the intraocular lenses [11, 21] have at their edge or on the haptic, parts such as holes, notches [8] or webs [14, 24] for rotating the lenses about their optical axis [A].

7. Lens arrangement according to one of the preceding claims, characterised in that the compensating ground region is a ground region with an optically cylindrical or toroidal effect for the correction of astigmatism.

8. Lens arrangement according to one of the preceding claims, characterised in that the material for the lens arrangement is a suitable soft or hard material, such as PMMA, silicon, hydrogel, foldable acrylate etc..

9. Lens arrangement according to one of the preceding claims, characterised in that at least one of the lenses [21] of the lens arrangement is a multifocal lens.

## Revendications

1. Agencement de lentilles intra-oculaires (1, 1') comportant, le long d'un axe optique commun, plusieurs lentilles intra-oculaires (11, 21) disposées l'une derrière l'autre, au moins une des lentilles intra-oculaires (11, 21) comportant, afin de corriger l'astigmatisme, un polissage optique (6) de compensation d'astigmatisme, caractérisé en ce que l'agencement de lentilles (1, 1') est constitué de deux lentilles intra-oculaires (11, 21) dont chacune comporte un polissage de compensation (6) adapté à la correction de l'astigmatisme ayant une orientation préférentielle linéaire (M, M1, M2), et en ce que les deux lentilles intra-oculaires (11, 21) peuvent tourner l'une par rapport à l'autre autour de l'axe optique commun (A) à l'état implanté.

2. Agencement de lentilles selon la revendication 1, caractérisé en ce que les deux lentilles intra-oculaires (11, 21) sont reçues dans un support annulaire permettant la rotation l'une par rapport à l'autre des lentilles intra-oculaires.

3. Agencement de lentilles selon la revendication 1 ou 2, caractérisé en ce que chacune des deux lentilles intra-oculaires (11, 21) est munie d'un support annulaire (12, 32) et en ce que les deux supports annulaires (12, 22) sont reçus à rotation dans une bague (30) commune implantable.

4. Agencement de lentilles selon la revendication 3, caractérisé en ce que la bague (30) présente, le long de son bord interne, deux gorges parallèles (31, 32) pour recevoir les supports annulaires (12, 13, 22, 23).

5. Agencement de lentilles selon l'une quelconque des revendications précédentes, caractérisé en ce que les lentilles intra-oculaires (11, 21) sont munies de repères (8) pour indiquer l'orientation linéaire préférentielle (M, M1, M2).

6. Agencement de lentilles selon l'une quelconque des revendications précédentes, caractérisé en ce que les lentilles intra-oculaires (11, 21) comportent sur leur bord ou sur une partie des supports annulaires, des évidements, des encoches (8) ou des nervures (14, 24) pour faire tourner les lentilles autour de leur axe optique (A).

7. Agencement de lentilles selon l'une quelconque des revendications précédentes, caractérisé en ce que le polissage de compensation, afin de réaliser une correction de l'astigmatisme, est un polissage à effet optique cylindrique ou torique.

8. Agencement de lentilles selon l'une quelconque des revendications précédentes, caractérisé en ce que le matériau de l'agencement de lentilles est un matériau adéquat mou ou dur tel que du PMMA, du silicone, de l'hydrogel, l'acrylate pliable, etc.

9. Agencement de lentilles selon l'une quelconque des revendications précédentes, caractérisé en ce qu'au moins l'une des lentilles (21) de l'agencement est une lentille à plusieurs foyers.
